# EUROPÄISCHE PATENTANMELDUNG

(11) **EP 2 308 427 A2**
(43) Veröffentlichungstag der Anmeldung: **13.04.2011**
(21) Anmeldenummer: 10009802.9
(22) Anmeldetag: 17.09.2010
(51) Int. Cl.: A61F 5/02

(54) **Orthopädisches Stützmittel**

(30) Priorität: 06.10.2009 DE 102009049542
(71) Anmelder: Bort GmbH, 71384 Weinstadt (DE)
(72) Erfinder: Bort, Wolfgang, 71334 Waiblingen (DE)
(74) Vertreter: Patentanwälte Ruff, Wilhelm, Beier, Dauster & Partner

(57) **Zusammenfassung**

1. Orthopädisches Stützmittel.
2.1. Die Erfindung betrifft ein orthopädisches Stützmittel (10) zur Stützung eines Körberbereichs (60a) eines Patienten mit mindestens einer Sensoreinrichtung, die zur Erfassung der Bewegung des Körperbereichs (60a) vorgesehen ist.
2.2. Erfindungsgemäß ist insbesondere vorgesehen, dass das Stützmittel (10) mindestens einen elastisch verformbaren Sensorabschnitt (1c - 8c) aufweist, der derart am Stützmittel angeordnet ist, dass eine Bewegung des Körperbereichs (60a) eine elastische Verformung des Sensorabschnitts (1c - 8c) bewirkt, dass die Sensoreinrichtung mindestens einen Aktor zur Einkopplung von Wellen in den Sensorabschnitt (1c - 8c) und mindestens einen Sensor zur Erfassung von in den Sensorabschnitt (1c - 8c) eingekoppelten Wellen aufweist, und dass der Aktor und der Sensor der Sensoreinrichtung derart am Sensorabschnitt (1c - 8c) angeordnet sind, dass der Sensor die durch den Aktor in den Sensorabschnitt (1c - 8c) eingekoppelte und in Abhängigkeit der Verformung des Sensorabschnitts (1c - 8c) veränderte Welle sensieren kann.
2.3. Verwendung zur Erfassung orthopädisch nachteiliger Bewegungen eines Patienten.
3. Fig. 5d.

## Beschreibung

Die Erfindung betrifft ein orthopädisches Stützmittel zur Stützung eines Körberbereichs eines Patienten. Ein gattungsgemäßes Stützmittel weist mindestens eine Sensoreinrichtung auf, die zur Erfassung der Bewegung des Körperbereichs vorgesehen ist.

Orthopädische Stützmittel dienen insbesondere der Wiederherstellung oder der Aufrechterhaltung der Funktionsfähigkeit von beschädigten oder besonders beanspruchten Körperteilen und Körperbereichen. Sie können als Bandagen ausgebildet sein, die überwiegend aus formflexiblem, beispielsweise textilem Material aufgebaut sind. Eine andere Variante der orthopädischen Stützmittel wird durch Orthesen gebildet, die zumindest zum Teil aus starren und nur begrenzt verformbaren Bestandteilen aufgebaut sind.

Aus dem Stand der Technik, beispielsweise der DE 10 2007 003 515 A1, ist es bekannt, Sensoren an einem orthopädischen Stützmittel vorzusehen, welche die Bewegung des zu stützenden Körperteils erfassen und eine orthopädisch ungünstige Bewegung gegebenenfalls durch eine Signalisierung für den Patienten erkennbar machen.

### Aufgabe und Lösung

Aufgabe der Erfindung ist es, ein gattungsgemäßes Stützmittel, insbesondere eine Rückenstützorthese, dahingehend weiterzubilden, dass in besonders zuverlässiger Weise die Bewegung des durch das Stützmittel gestützten Körperbereichs erfasst werden kann.

Erfindungsgemäß wird dies dadurch erreicht, dass das Stützmittel mindestens einen elastisch verformbaren Sensorabschnitt aufweist, der derart am Stützmittel angeordnet ist, dass eine Bewegung des Körperbereichs eine elastische Verformung dieses Sensorabschnitts bewirkt. Die Sensoreinrichtung umfasst weiterhin mindestens einen Aktor zur Einkopplung von Wellen in den Sensorabschnitt und mindestens einen Sensor zur Erfassung von in den Sensorabschnitt eingekoppelten Wellen. Der Aktor und der Sensor der Sensoreinrichtung sind derart am Sensorabschnitt angeordnet, dass der Sensor die durch den Aktor in den Sensorabschnitt eingekoppelte und in Abhängigkeit der Verformung des Sensorabschnitts veränderte Welle sensieren kann.

Bei dem erfindungsgemäßen Stützmittel ist somit vorgesehen, dass ein vorzugsweise flächiger Sensorabschnitt aus einem elastisch verformbaren Material derart positioniert ist, dass er infolge der Bewegung des Körperbereichs eine Verformung erfährt. Diese Verformung des Sensorabschnitts bewirkt einen Spannungszustand im Sensorabschnitt, die von der Art und dem Maß der Bewegung des Körperbereichs abhängt. Zur Erfassung dieses Spannungszustandes sind der Aktor und der Sensor der Sensoreinrichtung vorgesehen. Der Aktor koppelt eine Welle in den Sensorabschnitt ein, die sich im Sensorabschnitt fortpflanzt, bis sie den Sensor der Sensoreinrichtung erreicht. Zu diesem Zweck ist der Aktor derart ausgebildet, dass er durch eine Elektronik angeregt in Schwingung versetzt werden kann. Die in den Sensorabschnitt eingekoppelte Welle erfährt eine Veränderung, insbesondere in Hinblick auf ihre Amplitude, die davon beeinflusst ist, in welchem Maße der Sensorabschnitt verformt ist, also wie der im Sensorabschnitt herrschende Spannungszustand geartet ist. Die Veränderung wird für eine Elektronik der Bandage erfassbar, indem mittels des zur Erkennung von Schwingungen ausgebildeten Sensors die vom Sensor verursachte Welle sensiert wird.

Grundsätzlich können alle Formen von Wellenausbreitungen im Sensorabschnitt genutzt werden, beispielsweise auch Volumenwellen, die den Sensorabschnitt durchdringen. Als vorteilhaft wird es jedoch angesehen, wenn der Aktor dafür ausgebildet ist, Oberflächenwellen in den Sensorabschnitt einzukoppeln, und der Sensor dafür ausgebildet ist, diese Oberflächenwellen zu registrieren. Der Vorteil bei der Nutzung dieser Oberflächenwellen liegt darin, dass sie einer geringen Anregungsenergie zu ihrer Erzeugung bedürfen, was mit einer geringeren Betriebsspannung der Aktoren und somit einer Verminderung der Gefährdung des Patienten durch den genutzten elektrischen Strom einhergeht. Weiterhin ist der geringere Energiebedarf zur Einkopplung von Oberflächenwellen in Hinblick auf die Laufzeit mit einer Versorgungsbatterie von Vorteil. Die Verwendung von Oberflächenwellen gestattet darüber die Anordnung des Aktors und den Sensors auf der gleichen Seite des Sensorabschnitts, was konstruktive Vorteile mit sich bringen kann.

Der Aktor und der Sensor im Sinne dieser Erfindung stellen funktionale Begriffe dar. Neben einer Ausgestaltung, bei der Aktor und Sensor auch als Baukomponenten getrennt sind, ist auch eine Gestaltung mit nur einem Bauelement möglich, welches sowohl zum Abgeben als auch zum Empfangen von Wellen geeignet ist. Solche Bauelemente werden als Transceiver bezeichnet.

Als Aktor und Sensor der Sensoreinrichtung kommen solche Bauelemente in Frage, die zur Umwandlung einer elektrischen Spannung, insbesondere einer Wechselspannung, in mechanische Schwingungen bzw. einer mechanischen Schwingung in ein durch eine Elektronik auswertbares Signal geeignet sind. Insbesondere können der Aktor und/oder der Sensor als Piezo-Aktor bzw. Piezo-Sensor ausgebildet sein.

Durch die Anordnung des Sensorabschnitts und die Integration des Sensorabschnitts in das orthopädische Stützmittel kann vorgegeben werden, welche Art der Bewegung des Körperbereichs zu einer Veränderung der Welle und somit zu einer Registrierung einer Bewegung führt. Durch die Verwendung mehrerer Sensoreinrichtungen mit unterschiedlichen Sensorabschnitten können verschiedene Bereiche überwacht werden, so dass die Auswertung eine präzise Einschätzung der Bewegung des Patienten zulässt.

Vorzugsweise werden die Sensoreinrichtungen kontinuierlich ausgewertet. Der Sender der Sensoreinrichtung koppelt in einem solchen Fall kontinuierlich Wellen in den Sensorabschnitt ein. Es ist jedoch auch denkbar, mittels zeitlich beabstandeter Wellenimpulse im Abstand von einigen Hundertstel oder Zehntel Sekunden die Bewegung des Körperbereichs zu überwachen.

Bei einer Weiterbildung der Erfindung weist das Stützmittel mindestens einen Hauptabschnitt auf, der den Körperbereich derart umgibt, dass ein Bewegen des Körperbereichs eine Verformung des Hauptabschnitts bewirkt. Der Sensorabschnitt ist als zumindest abschnittsweise einstückiger Teil des Hauptabschnitts ausgebildet oder aber zumindest abschnittsweise durch einen am Hauptabschnitt angebrachten Zusatzabschnitt gebildet.

Der Hauptabschnitt bildet einen vorzugsweise einstückigen Abschnitt, der das Körperteil derart umgibt, dass ein Bewegen des Körperbereichs eine Verformung des Hauptabschnitts zur Folge hat. Im Falle einer Orthese kann ein solcher Hauptabschnitt als starres, jedoch begrenzt elastisch verformbares Teil ausgebildet sein. Im Falle einer Bandage kann der Hauptabschnitt auch unelastisch flexibel sein. Ein weitgehend starrer Hauptabschnitt ist vorzugsweise durch einen Überzug, beispielsweise einen vorzugsweise gepolsterten Textilüberzug, ergänzt, um den Tragekomfort zu erhöhen.

Im Falle eines starren, jedoch elastisch verformbaren Hauptabschnitts kann ein Teil dieses Hauptabschnitts unmittelbar den Sensorabschnitt der mindestens einen Sensoreinrichtung bilden. Dies führt zu einem sehr einfachen Aufbau.

Als bevorzugt wird jedoch die Verwendung eines Zusatzabschnitts angesehen, der am Hauptabschnitt kraftschlüssig, formschlüssig oder stoffschlüssig angebracht ist und somit bei einer Verformung des Hauptabschnitts mit verformt wird. Als besonders vorteilhaft wird die Verwendung stoffschlüssiger und/oder formschlüssiger Verbindungsmittel zur Festlegung des Zusatzabschnitts am Hauptabschnitt angesehen, insbesondere die Verwendung von Nieten. Die Verwendung eines vom Hauptabschnitt getrennten Zusatzabschnitts erlaubt es, jeweils optimale Materialien für den Hauptabschnitt und den Zusatzabschnitt zu verwenden. So kann der Hauptabschnitt beispielsweise als Kunststoffteil, insbesondere als Polyethylenteil ausgebildet sein, was mit geringen Herstellungskosten einhergeht. Der Zusatzabschnitt, der den Sensorabschnitt aufweist, kann dagegen in Hinblick auf ein möglichst idealelastisches Verhalten, insbesondere ein möglichst relaxationsfreies Verhalten, ausgebildet sein und hierfür insbesondere aus einem Faserverbundteil hergestellt sein. Insbesondere kohlenstofffaserverstärkter Kunststoff oder kohlenstofffaserverstärktes Epoxidharz eignen sich hierfür. Im Falle der Verwendung von Faserverbundwerkstoffen sind die Fasern vorzugsweise so auszurichten, dass sie in Richtung der vorgesehenen Wellenausbreitung vom Aktor zum Sensor verlaufen.

Im Falle einer Gestaltung mit einem separaten Zusatzabschnitt am Hauptabschnitt wird es als bevorzugt angesehen, wenn im Hauptabschnitt eine Ausnehmung vorgesehen ist, in der der Sensor und/oder der Aktor zumindest abschnittsweise angeordnet ist. Durch diese Gestaltung ist eine besonders flache Bauweise des orthopädischen Stützmittels zu erreichen, da der Sensor und/oder der Aktor auf der dem Hauptabschnitt zugewandten Seite des Zusatzabschnitts angeordnet sein können. Die Anordnung des Sensors und/oder des Aktors in der Ausnehmung kann durch insbesondere stoffschlüssige Anbindung des Aktors bzw. Sensors am Rand der Ausnehmung zusätzlich der Festlegung des Zusatzabschnitts am Hauptabschnitt dienen.

Eine Möglichkeit der Anordnung des Aktors und des Sensors zueinander sieht vor, dass Aktor und Sensor an gegenüberliegenden Enden des Sensorabschnitts mit diesem verbunden sind. Als vorteilhaft wird es jedoch angesehen, wenn ein Wellenreflektor am Sensorabschnitt vorgesehen ist, der dafür ausgebildet ist, eine vom Aktor kommende Welle in Richtung des Sensors zu reflektieren. Dies erlaubt es insbesondere, den Aktor und den Sensor der Sensoreinrichtung unmittelbar aneinander angrenzend zu positionieren oder sogar eine leicht handhabbare Baueinheit zu verwenden, die sowohl den Aktor als auch den Sensor umfasst.

Der Aktor und der Sensor der Sensoreinrichtung sind vorzugsweise so am Sensorabschnitt angeordnet, dass die durch den Aktor eingekoppelte Welle sich zumindest über einen Abstand von mindestens 5 mm ausbreitet, bis sie den Sensor erreicht. Im Falle eines Sensors und eines Aktors, die eine gemeinsame Baueinheit bilden, ist der Wellenreflektor dementsprechend vorzugsweise mindestens um 2,5 mm von dieser Baueinheit beabstandet. Bevorzugt werden größere Wellenausbreitungsdistanzen von mindestens 10 mm zwischen Aktor und Sensor.

Als Wellenreflektor wird eine Grenzfläche des Sensorabschnitts angesehen, die eine Reflektion der Welle bewirkt. Vorzugsweise wird der Wellenreflektor durch ein Befestigungsmittel wie beispielsweise eine Niete gebildet, welches der Festlegung des Zusatzabschnitts am Hauptabschnitt dient und somit eine Doppelfunktion übernimmt.

Ein erfindungsgemäßes orthopädisches Stützmittel kann der Stützung verschiedener Körperbereiche/Körperteile dienen und somit beispielsweise zur Verwendung am Kniegelenk oder dem Fußgelenk sowie am Ellenbogengelenk oder am Handgelenk ausgebildet sein. Auch ein Nackenstützmittel kann erfindungsgemäß ausgestaltet sein. Als besonders vorteilhaft wird es angesehen, wenn das orthopädische Stützmittel als Rückenstützorthese ausgebildet ist, wobei die Sensoreinrichtung vorzugsweise in einem Rückenbereich dieser Orthese angeordnet ist.

Die Erfassung des Spannungszustandes im Sensorabschnitt erfolgt vorzugsweise durch eine Auswertung der durch diesen Spannungszustand bewirkten Dämpfung der Welle. Die Anregungsfrequenz des Aktors ist dementsprechend vorzugsweise so zu wählen, dass keine zu starke Dämpfung, jedoch auch kein ungewolltes Aufschwingen bewirkt wird. Als besonders vorteilhaft wird es angesehen, wenn eine Steuerelektronik, die mit der Sensoreinrichtung verbunden ist, dafür ausgebildet ist, den Aktor mit einer Frequenz zwischen 10 kHz und 10 MHz anzuregen. Ein besonders gutes Signal-Rausch-Verhältnis wird in den Frequenzbereichen 25 kHz bis 100 kHz sowie 220 kHz bis 1 MHz erreicht.

Von Vorteil ist es, wenn ein erfindungsgemäßes orthopädisches Stützmittel mehrere Sensoreinrichtungen aufweist. Dabei ist es insbesondere von Vorteil, wenn die jeweiligen Sensorabschnitte von mindestens zwei dieser Sensoreinrichtungen durch einen einstückigen und am Hauptabschnitt angebrachten Zusatzabschnitt gebildet werden. Die Verwendung eines gemeinsamen Zusatzabschnitts, an dem mehrere Sensoreinrichtungen und somit mehrere Sensorabschnitte vorgesehen sind, führt zu einer einfacheren Bauweise, insbesondere zu einem verringerten Befestigungsaufwand zur Anbringung des Zusatzabschnitts am Hauptabschnitt.

Die Erfindung betrifft insbesondere als Weiterbildung des vorgenannten orthopädischen Stützmittels auch ein gattungsgemäßes orthopädisches Stützmittel, welches als Rückenstützorthese mit mindestens einem im Rückenbereich angeordneten Rückenabschnitt und mindestens einem mit dem Rückenabschnitt verbundenen Flügelabschnittspaar ausgebildet ist, wobei das Flügelabschnittspaar aus zwei seitlich am Rückenabschnitt und gegenüberliegend zueinander angebrachten Flügelabschnitten besteht, die sich um gegenüberliegende Körperseiten gebogen bis zum Brust- und/oder Bauchbereich des Patienten erstrecken. Dabei ist eine Mehrzahl von Sensoreinrichtungen vorgesehen, welche dafür ausgebildet sind, Längenänderungen oder Spannungszustände in dem orthopädischen Stützmittel zu erfassen, wobei mindestens eine Sensoreinrichtung einer ersten Art am Rückenabschnitt auf Höhe des Flügelabschnittpaares oder an einem der Flügelabschnitte angeordnet ist, und wobei mindestens eine Sensoreinrichtung einer zweiten Art am Rückenabschnitt unterhalb oder oberhalb des Flügelabschnitts angeordnet ist.

Die erfindungsgemäße Rückenorthese weist einen festen, jedoch begrenzt elastisch verformbaren Rückenabschnitt auf, von dem aus die Flügelabschnitte sich zur Brust- und Bauchseite des Patienten erstrecken. Die Flügelabschnitte sind dabei vorzugsweise einstückig mit dem Rückenabschnitt ausgebildet, zumindest aber an diesem festgelegt. Die Flügelabschnitte dienen zum einen der Festlegung der Rückenstützorthese am Körper des Patienten, wobei sie hierzu vorzugsweise im Bauchbereich oder Brustbereich des Patienten miteinander mittels lösbarer Verbindungsmittel wie Riemen und/oder Klettverschlüssen verbindbar sind. Zum anderen dienen die Flügelabschnitte zur Erkennung einer Bewegung des Torsos des Patienten. Hierfür sind die Sensoreinrichtungen der ersten und der zweiten Art vorgesehen.

Wenn der Patient seinen Oberkörper nach links oder rechts dreht, führt dies zu einer elastischen Verlagerung der Flügelabschnitte relativ zum Rückenabschnitt. Diese Verformung kann durch Erfassen einer entsprechenden Längenänderung oder eines Spannungszustandes durch die mindestens eine Sensoreinrichtung der ersten Art erfasst werden. Diese ist zu diesem Zweck entweder am Flügelabschnitt selbst angeordnet oder aber in einem Übergangsbereich zwischen einem Flügelabschnitt und dem Rückenabschnitt angeordnet, der sich bei einer elastischen Verlagerung des Flügelabschnitts gegenüber dem Rückenabschnitt partiell verformt. Zur Erfassung eines Vorbeugens oder Zurückbeugens des Patienten ist die mindestens eine Sensoreinrichtung der zweiten Art vorgesehen, die unterhalb oder oberhalb des Flügelabschnitts angeordnet ist. Wenn der Patient sich vorbeugt oder zurückbeugt, wird durch seinen Rücken oder aber mittels der Flügelabschnitte durch seine Brust oder seinen Bauch eine zumindest abschnittsweise Verformung des Rückenabschnitts der Orthese bewirkt, die durch diese Sensoreinrichtung der zweiten Art erfasst werden kann.

Die genannte Anbringung der Sensoreinrichtungen führt zu einer guten Unterscheidbarkeit einer Vorbeuge-/Zurückbeugebewegung von einer Drehbewegung. Eine Vorbeuge-/Zurückbeugebewegung bewirkt wegen des durch die Flügelabschnitte verursachten hohen mechanischen Widerstandsmoments im Bereich der Sensoreinrichtung der ersten Art kaum Spannungen bzw. Längenveränderungen im Bereich der Sensoreinrichtung der ersten Art. Eine solche Bewegung wird stattdessen vor allem durch die Sensoreinrichtung der zweiten Art erfasst, die oberhalb oder unterhalb der Flügelabschnitte angeordnet ist. Im Gegenzug wird durch eine Drehbewegung des Oberkörpers des Patienten im Wesentlichen nur einen Spannungszustand bzw. eine Längenveränderung auf Höhe der Flügelabschnitte und somit im Bereich der Sensoreinrichtung der ersten Art bewirkt, während die Sensoreinrichtung der zweiten Art hierdurch kaum beeinflusst wird.

Somit erfasst die Sensoreinrichtung der ersten Art insbesondere eine Biegeverformung der Orthese im Bereich der Flügelabschnitte, während die Sensoreinrichtung der zweiten Art insbesondere eine Biegeverformung im Bereich des Rückenabschnitts erfasst.

Es können statt einem Rückenabschnitt auch mehrere Rückenabschnitte vorgesehen sein, die beispielsweise durch Riemen miteinander verbunden sind. Vorzugsweise ist der Rückenabschnitt aber einteilig ausgebildet bzw. besteht aus mehreren Teilen, die fest miteinander verbunden sind.

Bei den Sensoreinrichtungen handelt es sich vorzugsweise um Sensoreinrichtungen der oben beschriebenen Gestaltung mit einem Aktor, einem Sensor und einem Sensorabschnitt, in den durch den Aktor eine Welle eingekoppelt werden kann, die in verformungsabhängig veränderter Form durch den Sensor erfassbar ist. Besonders von Vorteil ist es dabei, dass baugleiche Sensoreinrichtungen als Sensoreinrichtungen der ersten und der zweiten Art verwendet werden können, da sich sowohl die Drehbewegung des Patienten als auch Vorbeuge-/Zurückbeugebewegungen jeweils in Form von detektierbaren Spannungszuständen niederschlagen. Es ist sowohl möglich, die verschiedenen Aktoren der Sensoreinrichtungen mit unterschiedlichen als auch mit der gleichen Frequenz zu betreiben. Letzteres gestattet ein hinsichtlich der Leitungsverlegung besonders einfache Parallelschaltung der Aktoren.

Neben Sensoreinrichtungen auf Basis von Wellenauswertung können auch andere Sensoren Verwendung finden, insbesondere zur Abstandsmessung ausgebildete Sensoren wie Dehnungsmessstreifen oder optische Abstandssensoren.

Vorzugsweise sind zwei Sensoreinrichtungen der ersten Art vorgesehen, die jeweils einem Flügelabschnitt eines gemeinsamen Flügelabschnittpaares zugeordnet sind. Die Sensoreinrichtungen können in der beschriebenen Weise entweder im Übergangsbereich zwischen dem Rückenabschnitt und dem Flügelabschnitt vorgesehen sein oder am Flügelabschnitt selbst vorgesehen sein. Durch die Gestaltung mit zwei solchen Sensoreinrichtungen der ersten Art ist es möglich, zu erfassen, in welche Richtung ein Patient seinen Oberkörper dreht.

Weiterhin sind vorzugsweise zwei vertikal gegeneinander beabstandete Flügelabschnittspaare vorgesehen, wobei insbesondere vorzugsweise mindestens zwei Sensoreinrichtungen der ersten Art oder mindestens zwei Paar Sensoreinrichtungen der ersten Art vorgesehen sind, die jeweils einem der Flügelabschnittspaare zugeordnet sind. Die Verwendung zweier Flügelabschnittspaare ist insbesondere deshalb von Vorteil, da hierdurch die mindestens eine Sensoreinrichtung der zweiten Art besonders zuverlässig ein Vorbeugen des Patienten erkennen kann, da die voneinander beabstandeten Flügelabschnittspaare zu einer Biegeverformung des Rückenabschnitts führen, wenn der Patient sich vorbeugt. Eine Gestaltung mit jeweils an den Flügelabschnittspaaren vorgesehenen Sensoreinrichtungen der ersten Art gestattet eine besonders zuverlässige Registrierung einer Oberkörperdrehung des Patienten.

Weiterhin wird es als besonders bevorzugt angesehen, wenn mindestens zwei Sensoreinrichtungen der zweiten Art am Rückenabschnitt vorgesehen sind, von denen eine erste Sensoreinrichtung in vertikaler Richtung zwischen zwei Flügelabschnittspaaren angeordnet ist und von denen eine zweite Sensoreinrichtung oberhalb des obersten Flügelabschnittspaares oder unterhalb des untersten Flügelabschnittspaares angeordnet ist. Diese Gestaltung mit mindestens zwei Sensoreinrichtungen der zweiten Art, die vertikal gegeneinander versetzt sind, ist in Hinblick auf die Unterscheidbarkeit einer Vorbeugebewegung von einer Zurückbeugebewegung von Vorteil. Während beim Zurückbeugen aufgrund der Verformung des Rückens des Patienten alle Sensoreinrichtungen der zweiten Art eine Längenänderung oder ein Spannungszustand erfassen, gilt dies beim Vorbeugen nur für jene Sensoreinrichtungen, die zwischen Flügelabschnittspaaren angeordnet sind. Die Sensoreinrichtung der zweiten Art, die oberhalb des obersten Flügelabschnittspaares oder unterhalb des untersten Flügelabschnittspaares angeordnet ist, erfährt beim Vorbeugen keine oder eine deutlich schwächere Einflussnahme, so dass insbesondere anhand dieser derart angeordneten zweiten Sensoreinrichtung die Unterscheidung zwischen Vorbeugen und Zurückbeugen zuverlässig möglich ist.

Ein erfindungsgemäßes Stützmittel weist vorzugsweise eine Auswerteelektronik auf, die mit der mindestens einen Sensoreinrichtung bzw. mit den mindestens zwei Sensoreinrichtungen verbunden ist und die in Abhängigkeit der Signale dieser Sensoreinrichtung mittels einer Signalisierungseinrichtung eine orthopädisch nachteilige Bewegung des Körperbereichs signalisiert. Die Auswerteelektronik wertet hierzu die von den Sensoreinrichtungen kommenden Signale aus und schließt dabei auf Basis des Signals oder der im Falle von mehreren Sensoreinrichtungen mehreren Signalen auf die vom Patienten durchgeführte Bewegung. Diese Bewegung wird im einfachsten Falle als orthopädisch zulässig oder orthopädisch nachteilig klassifiziert. Eine orthopädisch nachteilige Bewegung führt zur Abgabe eines Patienteninformationssignals durch die Signalisierungseinrichtung, beispielsweise in Form eines Warntons oder einer Vibration. Die Auswerteelektronik kann auch dafür ausgebildet sein, Kenndaten der vom Patienten durchgeführten Bewegungen zum Teil oder vollständig in einem Speicher abzulegen, so dass ein Orthopäde diese Daten später auswerten kann.

Im Zusammenhang mit den Sensoreinrichtungen auf Basis einer Wellenauswertung umfasst die Auswertung vorzugsweise die Auswertung der Dämpfung der Welle und/oder der Laufzeit der Welle. Um die Dämpfung der Welle einfacher auswerten zu können, kann der Auswertelektronik eine Schaltung vorgeschaltet sein, die das von der Sensoreinrichtung stammende Signal zu einem transformierten Signal umwandelt, dessen Signalstärke von der Amplitude des von der Sensoreinrichtung stammende Signals abhängt. Eine solche Schaltung kann beispielsweise so geartet sein, dass sie das von der Sensoreinrichtung stammende Signal umrichtet und anschließend tiefpassfiltert. Die Umwandlung reduziert die Komplexität der Auswerteelektronik, insbesondere die erforderliche Leistung eines Mikroprozessors der Auswerteelektronik, da nicht die durch den Sensor erfasste Welle selbst, sondern nur deren Amplitude durch die Auswerteelektronik ausgewertet werden muss. Zur Ermittlung der jeweiligen Dämpfung der Welle durch den Spannungszustand im Sensorabschnitt ist dies ausreichend.

Die jeweiligen Grenzwerte, ab denen die Auswerteelektronik einen orthopädisch ungünstigen Zustand erkennt und gegebenenfalls über die Signalisierungseinrichtung kenntlich macht, können fest vorgegeben oder gezielt einstellbar sein. Von besonderem Vorteil ist es jedoch, wenn die Auswerteelektronik in ein Initialisierungsmodus überführbar ist, indem die Grenzwerte in Abhängigkeit von Signalen der Sensoreinrichtung gebildet werden, wobei die Auswerteelektronik dafür ausgebildet ist, diese Grenzwerte dann in einen nachfolgenden Betriebsmodus zur Erkennung orthopädisch nachteiliger Bewegung des Körperbereichs zu nutzen. Der Initialisierungsmodus, der beispielsweise über eine spezielle Taste an dem Stützmittel gestartet werden kann, erlaubt die bequeme Anpassung des Stützmittels an einen individuellen Patienten. Während der Initialisierungsmodus läuft, bewegt sich der Patient gegebenenfalls gemäß einer mitgelieferten Bedienungsanleitung, unter Anleitung eines Orthopäden oder unter Anleitung der Steuerelektronik selbst derart, dass die Auswerteelektronik erfassen kann, zu welchen Signalen der Sensoren es bei vorgegebenen Bewegungen des spezifischen Körperbereichs/Körperteils kommt. Die anhand dessen festgelegten Grenzwerte sind somit individuell auf den Patienten zugeschnitten.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches anhand der Figuren erläutert wird. Dabei zeigen:
- Fig. 1 bis 3: eine erfindungsgemäße Rückenstützorthese in einer An- sicht von hinten und von vorne sowie in einer geschnitte- nen Ansicht von oben,
- Fig. 4a bis 4c: die Funktionsweise der Sensoreinrichtungen der Rü- ckenstützorthese gemäß der Fig. 1 bis 3 und
- Fig. 5a bis 5d: die Nutzung der Rückenstützorthese der Fig. 1 bis 3 und die mit verschiedenen Bewegungen eines Patienten ein- hergehenden Beeinflussungen der Sensoreinrichtungen der Orthese.

### Detaillierte Beschreibung des Ausführungsbeispiels

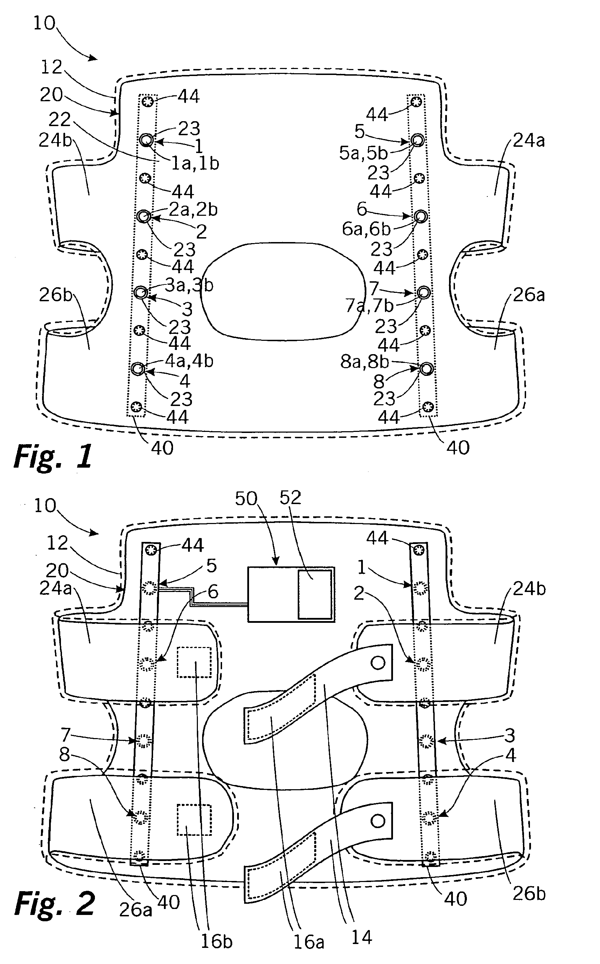

Die Fig. 1 bis 3 zeigen eine erfindungsgemäße Rückenstützorthese 10.

Die Rückenstützorthese 10 umfasst einen Hauptabschnitt 20, der als einstückiges flächiges Kunststoffteil aus Polyethylen oder einem anderen Kunststoff gefertigt ist. Dieser Hauptabschnitt 20 umfasst einen Rückenabschnitt 22, an dem seitlich ein oberes Flügelabschnittspaar 24 mit Flügelabschnitten 24a, 24b und ein unteres Flügelabschnittspaar 26 mit den Flügelabschnitten 26a, 26b vorgesehen sind. Diese Flügelabschnitte 26a, 26b, 24a, 24b sind auf gegenüberliegenden Seiten beidseitig des Rückenabschnitts 22 an diesen angeformt und erstrecken sich bogenförmig nach vorne.

Der Hauptabschnitt 20 der Rückenstützorthese 10 ist vollständig von einer textilen Hülle 12 umgeben, die in den Darstellungen der Fig. 1 bis 3 gestrichelt dargestellt ist. Ihr Zweck liegt insbesondere in einer Erhöhung des Tragekomforts.

An der Vorderseite der Rückenstützorthese 10 sind an den Flügelabschnitten 24b, 26b jeweils Verschlussriemen 14 angebracht, an deren freiem Ende eine Klettfläche 16a vorgesehen ist, die zum Zusammenwirken mit jeweils korrespondierenden Klettflächen 16b an den Flügelabschnitten 24a, 26a vorgesehen ist und so ein Verbinden der Flügelabschnitte 24a, 24b, 26a, 26b vor dem Bauch bzw. der Brust des Patienten gestattet.

Beidseitig sind seitlich am Rückenabschnitt 22 zwei im Wesentlichen vertikal erstreckte Flachstäbe 40 aus CFK vorgesehen, der mittels Nieten 44 mit dem Hauptabschnitt 20 verbunden ist. Diese Flachstäbe 40 sind auf der Innenseite des Rückenabschnitts 22 vorgesehen, jedoch durch die textile Hülse 12 derart abgedeckt, dass sie für den Patienten nicht zu sehen sind.

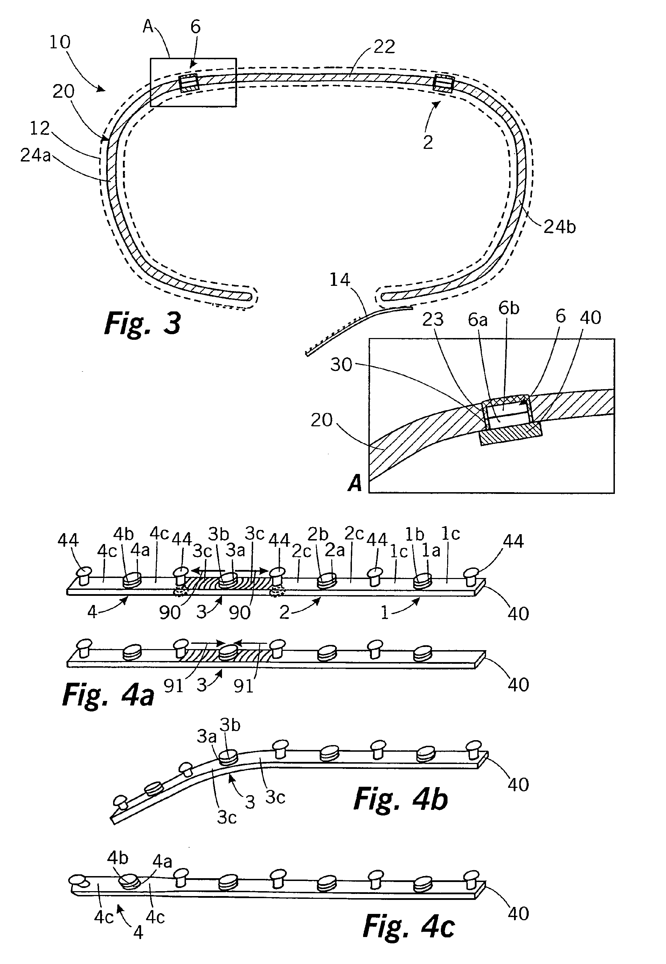

Die Flachstäbe 40 dienen als Träger für Sensoreinrichtungen 1, 2, 3, 4, 5, 6, 7, 8. Bezugnehmend insbesondere auch auf Fig. 3 sowie den in Fig. 3 dargestellten vergrößerten Teilbereich A umfassen die Sensoreinrichtungen jeweils einen Aktor 1a - 8a und einen Sensor 1b - 8b. Diese Aktoren 1a - 8a und diese Sensoren 1b - 8b sind unmittelbar aneinander anliegend vorgesehen. Die Aktoren 1a - 8a sind darüber hinaus unmittelbar an den Flachstäben 40 angebracht, beispielsweise mittels einer Klebeverbindung. Die Aktoren 1a - 8a sind als Piezo-Aktoren ausgebildet, die entsprechend angeregt hochfrequent schwingen können. Die Sensoren 1b - 8b sind als Piezo-Sensoren dafür ausgebildet, Schwingungen erfassen zu können und sie in ein auswertbares elektrisches Signal zu überführen.

Wie sich anhand der Detaildarstellung A der Fig. 3 ergibt, sind die Aktoren 1a - 8a und die Sensoren 1b - 8b in Ausnehmungen 23 des Rückenabschnitts 22 des Hauptabschnitts 20 angeordnet. Sie sind dort mittels eines Klebstoffs 30 verklebt, so dass die Flachstäbe 40 nicht nur über die Nieten 44, sondern auch über die Sensoren 1b - 8b und die Aktoren 1a - 8a sowie über den Klebstoff 30 mit dem Hauptkörper 20 mechanisch verbunden sind.

Am Rückenabschnitt 22 ist verdeckt durch die textile Hülse 12 weiterhin ein Steuer- und Auswertegerät 50 an der Innenseite des Rückenabschnitts 22 angeordnet, welches mit den Aktoren 1a - 8a und den Sensoren 1b - 8b über Leitungen verbunden ist. Dies ist lediglich beispielhaft in Fig. 2 für die Sensoreinrichtung 5 verdeutlicht. Die Anbindung der anderen Sensoreinrichtungen an das Steuergerät 50 ist entsprechend gestaltet.

Das Steuergerät 50 ist dafür ausgebildet, die Aktoren 1a - 8a mit einer hochfrequenten Wechselspannung zu versorgen, um eine Schwingung dieser Aktoren zu bewirken. Dabei findet eine Frequenz von 500 kHz Anwendung. Weiterhin ist das Steuergerät 50 dafür ausgebildet, die durch die Sensoren 1 b bis 8b erfassten Schwingungen auszuwerten.

Anhand der Fig. 4a bis 4c wird nachfolgend die Funktionsweise der Sensoreinrichtungen 1 - 8 erläutert. Dies erfolgt anhand der in Fig. 1 linksseitigen Sensoreinrichtungen 1 bis 4.

Wie bereits erläutert, sind die Sensoreinrichtungen 1 - 4 an einem der beiden CFK-Flachstäbe 40 vorgesehen. Jedem Set aus einem Aktor der Aktoren 1a bis 8a sowie einem Sensor der Sensoren 1b bis 8b ist ein Sensorabschnitt 1c, 2c, 3c, 4c zugeordnet. Bei diesem Sensorabschnitt 1c, 2c, 3c, 4c handelt es sich jeweils um einen Teilabschnitt des Flachstabs 40, wobei jeder Sensorabschnitt beidseitig durch jeweils eine Niete 44 begrenzt wird. Der oberhalb des Flachstabs 40 angeordnete Hauptkörper 20 ist in der Darstellung der Fig. 4a bis 4c ausgeblendet.

Anhand der Fig. 4a und am Beispiel der Sensoreinrichtung 3 wird die Funktionsweise der Sensoreinrichtungen 1 bis 8 erläutert. Der Aktor 3a wird vom Steuergerät 50 mit einer Wechselspannung mit der genannten Frequenz von 500 kHz angeregt. Die dadurch bewirkte Schwingung des Aktors 3a führt zu einer Welleneinkopplung in den Sensorabschnitt 3c. Die Wellen breiten sich in beide Richtungen 90 aus, bis sie von den Nieten 44 in Richtung 91 reflektiert werden. Die zurückgeworfenen Wellen werden von dem Sensor 3b erfasst und als entsprechendes Signal an das Steuergerät 50 gesendet. Das Steuergerät 50 kann die registrierte Welle mit der eingekoppelten Welle vergleichen und dementsprechend beispielsweise anhand der Dämpfung Rückschlüsse auf den Spannungszustand im Sensorabschnitt 2c ziehen.

Im Zustand der Fig. 4a befindet sich der Flachstab 40 im entspannten Zustand, so dass die Dämpfung gering ist.

Im Zustand der Fig. 4b ist der Sensorabschnitt 3c der Sensoreinrichtung 3 durch eine Kraftbeaufschlagung des Flachstabes 40 gebogen und steht somit an der Oberfläche unter Zugspannung. Die vom Aktor 3a in den Sensorabschnitt 3c eingekoppelte Wellen werden aufgrund dieses Spannungszustandes stärker gedämpft, bevor sie vom Sensor 3b erfasst werden. Das Steuergerät 50 ist somit in der Lage, aufgrund der erhöhten Dämpfung den Spannungszustand und somit die Verformung des Sensorabschnitts 3c zu erkennen.

Im Zustand der Fig. 4c ist der Flachstab 40 im Bereich des Sensorabschnitts 4c tordiert. Auch dies führt zu einem Spannungszustand, der eine vom Aktor 4a in den Sensorabschnitt 4c eingekoppelte Welle in gegenüber dem spannungsfreien Zustand der Fig. 4a stärker gedämpfter Form am Sensor 4b ankommen lässt.

Obwohl die Spannungszustände des Sensorbereichs 3c in Fig. 4b und des Sensorbereichs 4c in Fig. 4c zum einen durch eine Biegung und zum anderen durch eine Tordierung bewirkt werden, können sie durch baugleiche Sensoren/Aktoren erkannt werden, da beide Arten von Spannungszuständen eine verstärkte Dämpfung bewirken.

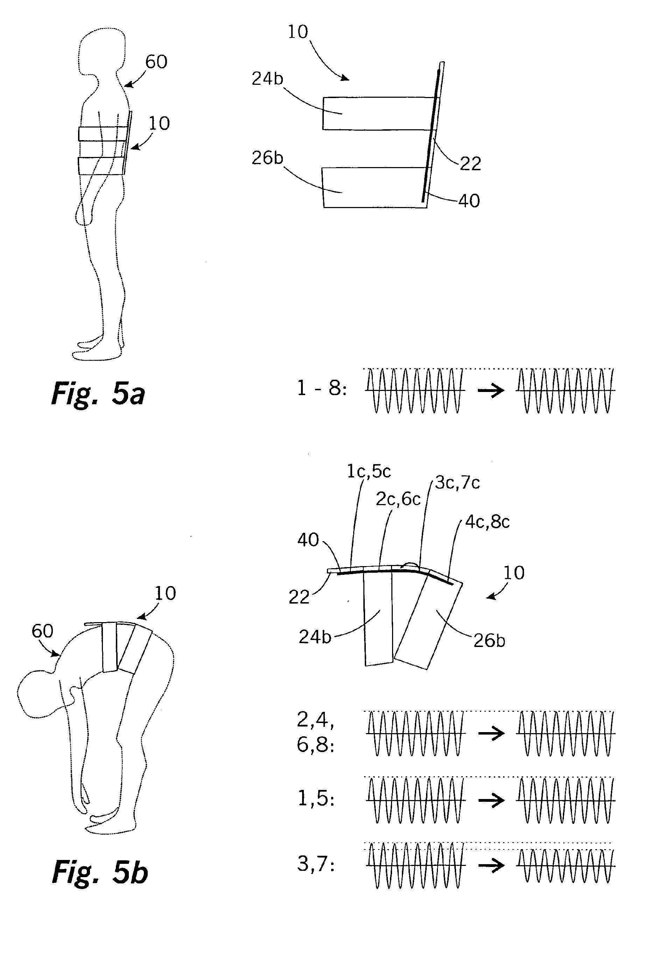

Die bewegungsspezifische Verformung der Flachstäbe 40 bei einer Verformung des Hauptabschnitts 22 wird genutzt, um verschiedene Bewegungen des Patienten durch das Steuergerät 50 erkennbar und unterscheidbar zu machen. Anhand der Fig. 5a bis 5d wird nachfolgend in vereinfachter Form erläutert, wie dies vonstatten geht.

Fig. 5a zeigt einen Ausgangszustand der Rückenorthese 10. Der Patient 60 befindet sich in einer aufrechten Haltung. Die Flachstäbe 40 sind in diesem Zustand entsprechend der Darstellung der Fig. 4a entspannt. Dies führt dazu, dass die in Fig. 5a links dargestellte eingekoppelte Welle nahezu ungedämpft die jeweiligen Sensoren 1b - 8b erreicht, wie anhand der Schwingungsdarstellungen der Fig. 5a ersichtlich ist. Das Steuergerät erkennt hierdurch die aufrechte Haltung des Patienten 60.

Fig. 5b zeigt den Patienten 60 in nach vorne gebeugter Haltung. Diese Haltung bewirkt aufgrund der Flügelabschnitte 24a, 24b, 26a, 26b, dass die Flachstäbe 40 im Bereich der Sensorabschnitte 3c, 7c der Sensoreinrichtungen 3, 7 gebogen wird. Die übrigen Sensorabschnitte 1c, 2c, 4c, 5c, 6c, 8c werden nicht oder kaum gebogen. Im Falle der Sensorabschnitte 1c, 5c liegt dies daran, dass diese noch oberhalb der oberen Flügelabschnitte 24a, 24b vorgesehen sind, so dass die Flügelabschnitte 24a, 24b, 26a, 26b hier kein Biegemoment erzeugen können. Im Falle der Sensorabschnitte 2c, 4c, 6c, 8c liegt es an dem durch die Flügelabschnitte 24a, 24b, 26a, 26b erhöhten mechanischen Widerstandsmoment des Rückenabschnitts 22 gegen Verbiegen im Bereich dieser Sensorabschnitte. Wie die Wellendarstellung der Fig. 5b zeigen, ist eine erhöhte Dämpfung daher nur im Bereich der Sensoreinrichtungen 3 und 7 zu verzeichnen. Eine derartige auf die Sensoreinrichtungen 3, 7 beschränkte Dämpfung kann von dem Steuergerät 50 somit deutlich als Zeichen einer Vorbeugebewegung erkannt werden.

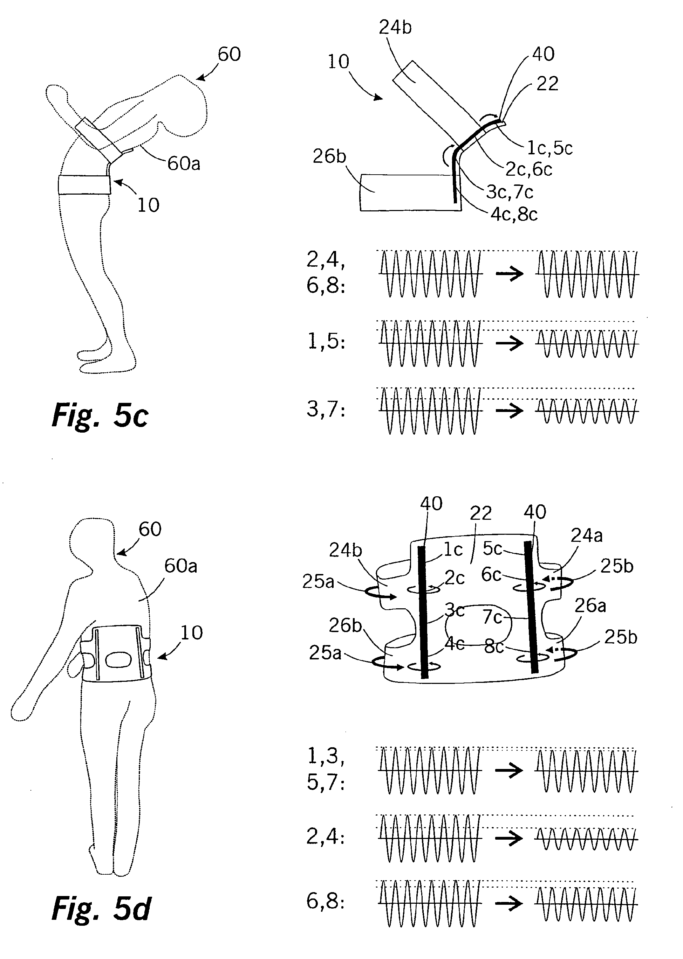

Die Fig. 5c zeigt den Patienten 60 in einer zurückgebeugten Haltung. Auch diese Haltung führt zu einem Verbiegen des Rückenabschnitts 22 und damit der Flachstäbe 40. Anders als bei der vorgebeugten Haltung der Fig. 5b wird dies jedoch durch die Haltung des Rückens 60a des Patienten bewirkt, so dass abweichend vom Vorbeugen der Fig. 5b auch die Sensoreinrichtungen 1, 5 eine erhöhte Dämpfung registrieren. Die Sensoreinrichtungen 2, 4, 6, 8 hingegen bleiben wiederum aufgrund des erhöhten Widerstandsmomentes des Rückenabschnitts 22 durch die Flügelabschnitte 24a, 24b, 26a, 26b weitgehend unbeeinflusst. Die erfassten Dämpfungen können vom Steuergerät 50 somit klar einer Zurückbeugebewegung zugeordnet werden.

Fig. 5d zeigt eine Stellung des Patienten, in der dieser seinen Oberkörper nach links verdreht hat. Eine solche Bewegung bewirkt, dass die linksseitigen Flügelabschnitte 24b, 26b in Richtung der Pfeile 25a nach hinten gebogen werden. Gleichzeitig werden über die Verschlussriemen 14 die rechtsseitigen Flügelabschnitte 24a, 26a an der Vorderseite des Patienten nach links gezogen, so dass sie in Richtung der Pfeile 25b nach vorne gebogen werden. Diese Biegebeanspruchung der Flügelabschnitte 24a, 24b, 26a, 26b führt dazu, dass die Flachstäbe 40 jeweils in Richtung der dargestellten Pfeile partiell im Bereich der Sensorabschnitte 2c, 4c, 6c, 8c tordiert werden, so dass die Sensoreinrichtungen 2, 4, 6, 8 eine erhöhte Dämpfung registrieren. Aufgrund der Tatsache, dass diese Torsion in Drehrichtung, also linksseitig, stärker als rechtsseitig auftritt, kann durch das Steuergerät 50 erfasst werden, dass es sich um eine Körperbewegung nach links handelt.

Aus den Erläuterungen zu den Fig. 5a bis 5d ist ersichtlich, dass das Steuergerät 50 die Bewegung des Patienten zuverlässig erfassen kann. Durch das Maß der Dämpfung lässt sich nicht nur die Art, sondern auch das Maß der jeweiligen Bewegung erfassen. Das Steuergerät 50 kann die ermittelte Bewegung bzw. Haltung des Patienten mit vorgegebenen Grenzwerten vergleichen, um beurteilen zu können, ob es sich um eine orthopädisch nachteilige Bewegung bzw. Haltung handelt. Wenn dies der Fall ist, so kann das Steuergerät 50 über einen integrierten Vibrationssignalgeber 52 den Patienten darüber informieren, dass seine gegenwärtige Bewegung bzw. Haltung vermieden werden sollte.

## Patentansprüche

1. Orthopädisches Stützmittel (10) zur Stützung eines Körberbereichs (60a) eines Patienten mit
- mindestens einer Sensoreinrichtung (1 - 8), die zur Erfassung der Bewegung des Körperbereichs (60a) vorgesehen ist,
**dadurch gekennzeichnet, dass**
- das Stützmittel (10) mindestens einen elastisch verformbaren Sensorabschnitt (1c - 8c) aufweist, der derart am Stützmittel angeordnet ist, dass eine Bewegung des Körperbereichs (60a) eine elastische Verformung des Sensorabschnitts (1c - 8c) bewirkt,
- die Sensoreinrichtung (1 - 8) mindestens einen Aktor (1a - 8a) zur Einkopplung von Wellen in den Sensorabschnitt (1c - 8c) und mindestens einen Sensor (1b - 8b) zur Erfassung von in den Sensorabschnitt (1c - 8c) eingekoppelten Wellen aufweist, und
- der Aktor (1 a - 8a) und der Sensor (1b - 8b) der Sensoreinrichtung derart am Sensorabschnitt (1c - 8c) angeordnet sind, dass der Sensor (1b - 8b) die durch den Aktor (1a - 8a) in den Sensorabschnitt (1c - 8c) eingekoppelte und in Abhängigkeit der Verformung des Sensorabschnitts (1c - 8c) veränderte Welle sensieren kann.

2. Orthopädisches Stützmittel nach Anspruch 1,
**dadurch gekennzeichnet, dass**
- das Stützmittel (10) mindestens einen Hauptabschnitt (20) aufweist, der den Körperbereich (60a) derart umgibt, dass eine Bewegung des Körperbereichs (60a) eine Verformung des Hauptabschnitts (20) bewirkt, und
- der Sensorabschnitt zumindest abschnittsweise einstückiger Teil des Hauptabschnitts ist und/oder der Sensorabschnitt (1c - 8c) zumindest abschnittsweise durch einen am Hauptabschnitt (20) angebrachten Zusatzabschnitt (40) gebildet wird.

3. Orthopädisches Stützmittel nach Anspruch 2,
**dadurch gekennzeichnet, dass**
der Hauptabschnitt (20) als Kunststoffteil, insbesondere als Polyethylenteil, ausgebildet ist und/oder der Zusatzabschnitt (40) aus einem Faserverbundwerkstoff hergestellt ist, insbesondere aus CFK.

4. Orthopädisches Stützmittel nach Anspruch 2 oder 3,
**dadurch gekennzeichnet, dass**
im Hauptabschnitt (20) eine Ausnehmung (23) vorgesehen ist, in der der Sensor (1b - 8b) und/oder der Aktor (1a - 8a) zumindest abschnittsweise angeordnet sind, wobei der Aktor (1a - 8a) bzw. der Sensor (1b - 8b) in der Ausnehmung (23) vorzugsweise eingegossen oder eingeklebt (30) sind.

5. Orthopädisches Stützmittel nach einem der vorstehenden Ansprüche,
**dadurch gekennzeichnet, dass**
ein Wellenreflektor (44) am Sensorabschnitt (1c - 8c) vorgesehen ist, der dafür ausgebildet ist, eine vom Aktor (1a - 8a) kommende Welle in Richtung des Sensors (1b - 8b) zu reflektieren, wobei vorzugsweise der Aktor (1a - 8a) und der Sensor (1b - 8b) der Sensoreinrichtung unmittelbar aneinander angrenzend positioniert sind.

6. Orthopädisches Stützmittel nach einem der vorstehenden Ansprüche,
**dadurch gekennzeichnet, dass**
das Stützmittel (10) als Rückenstützorthese (10) ausgebildet ist, wobei die Sensoreinrichtung (1 - 8) vorzugsweise an einem Rückenbereich (22) der Orthese (10) angeordnet ist.

7. Orthopädisches Stützmittel nach einem der vorstehenden Ansprüche,
**gekennzeichnet durch**
eine Steuerelektronik (50), die mit der Sensoreinrichtung (1 - 8) verbunden ist, wobei diese Steuerelektronik (50) dafür ausgebildet ist, den Aktor (1a - 8a) mit einer Frequenz zwischen 10 kHz und 10 MHz anzuregen, insbesondere mit einer Frequenz zwischen 25 kHz und 100 kHz oder mit einer Frequenz zwischen 220 kHz und 1 MHz.

8. Orthopädisches Stützmittel nach einem der vorstehenden Ansprüche,
**dadurch gekennzeichnet, dass**
eine Mehrzahl von Sensoreinrichtungen (1 - 8) vorgesehen ist, wobei die jeweiligen Sensorabschnitte (1c - 8c) von mindestens zwei dieser Sensoreinrichtungen (1 - 8) durch einen einstückigen und am Hauptabschnitt (20) angebrachten Zusatzabschnitt (40) gebildet werden.

9. Orthopädisches Stützmittel nach einem der vorstehenden Ansprüche oder nach dem Oberbegriff von Anspruch 1,
**dadurch gekennzeichnet, dass**
- das Stützmittel (10) als Rückenstützorthese (10) mit mindestens einem im Rückenbereich angeordneten Rückenabschnitt (22) und mindestens einem mit dem Rückenabschnitt (22) verbundenen Flügelabschnittspaar (24, 26) ausgebildet ist, wobei das Flügelabschnittspaar (24, 26) aus zwei seitlich am Rückenabschnitt und gegenüberliegend zueinander angebrachten Flügelabschnitten (24a, 24b, 26a, 26b) besteht, die sich um gegenüberliegende Körperseite gebogen bis zum Brust- und/oder Bauchbereich erstrecken, und
- eine Mehrzahl von Sensoreinrichtungen (1 - 8) vorgesehen ist, die dafür ausgebildet sind, Längenänderungen oder Spannungszustände in dem orthopädischen Stützmittel zu erfassen, wobei
- mindestens eine Sensoreinrichtung (2, 4, 6, 8) einer ersten Art am Rückenabschnitt (22) auf Höhe des Flügelabschnittpaars (24, 26) oder an einem der Flügelabschnitte (24a, 24b, 26a, 26b) angeordnet ist und
- mindestens eine Sensoreinrichtung (1, 3, 5, 7) einer zweiten Art am Rückabschnitt (22) unterhalb oder oberhalb des Flügelabschnittspaars (24, 26) angeordnet ist.

10. Orthopädisches Stützmittel nach Anspruch 9,
**dadurch gekennzeichnet, dass**
zwei Sensoreinrichtungen (2, 4, 6, 8) der ersten Art vorgesehen sind, die jeweils einem Flügelabschnitt (24a, 24b, 26a, 26b) eines gemeinsamen Flügelabschnittspaares (24, 26) zugeordnet sind.

11. Orthopädisches Stützmittel nach Anspruch 9 oder 10,
**dadurch gekennzeichnet, dass**
zwei vertikal gegeneinander beabstandeten Flügelabschnittspaaren (24, 26) vorgesehen sind, wobei vorzugsweise mindestens zwei Sensoreinrichtungen (2, 4, 6, 8) der ersten Art oder mindestens zwei Paar Sensoreinrichtungen der ersten Art vorgesehen sind, die jeweils einem der Flügelabschnittspaare (24, 26) zugeordnet sind.

12. Orthopädisches Stützmittel nach einem der Ansprüche 9 bis 11,
**dadurch gekennzeichnet, dass**
mindestens zwei Sensoreinrichtungen (1, 3, 5, 7) der zweiten Art am Rückenabschnitt (22) vorgesehen sind, von denen eine erste Sensoreinrichtung (3, 7) in vertikaler Richtung zwischen zwei Flügelabschnittspaaren (24, 26) angeordnet ist und von denen eine zweite Sensoreinrichtung (1, 5) oberhalb des obersten Flügelabschnittspaares (24) oder unterhalb des untersten Flügelabschnittspaares angeordnet ist.

13. Orthopädisches Stützmittel nach einem der vorstehenden Ansprüche,
**dadurch gekennzeichnet, dass**
eine Auswerteelektronik (50) vorgesehen ist, die mit der mindestens einen Sensoreinrichtung (1 - 8) verbunden ist und in Abhängigkeit der Signale der mindestens einen Sensoreinrichtung (1 - 8) mittels einer Signalisierungseinrichtung (52) eine orthopädisch nachteilige Bewegung des Körperbereichs (60a) signalisiert.

14. Orthopädisches Stützmittel nach Anspruch 13,
**dadurch gekennzeichnet, dass**
der Auswerteelektronik (50) eine Schaltung vorgeschaltet ist, die das von der Sensoreinrichtung (1 - 8) stammende Signal zu einem transformierten Signal umwandelt, dessen Signalstärke von der Amplitude des von der Sensoreinrichtung stammenden Signals abhängt.

15. Orthopädisches Stützmittel nach einem der vorstehenden Ansprüche,
**dadurch gekennzeichnet, dass**
die Auswerteelektronik (50) in einen Initialisierungsmodus überführbar ist, in dem Grenzwerte in Abhängigkeit von Signalen der Sensoreinrichtungen (1 - 8) gebildet werden, wobei die Auswerteelektronik dafür ausgebildet ist, die Grenzwerte in einem nachfolgenden Betriebsmodus zur Erkennung orthopädisch nachteiliger Bewegungen des Körperbereichs (60a) zu nutzen.
